# EUROPEAN PATENT APPLICATION

(11) **EP 4 260 868 A1**
(43) Date of publication of application: **18.10.2023**
(21) Application number: 21901803.3
(22) Date of filing: 13.01.2021
(51) Int. Cl.: A61K 38/17, C07K 14/435, A61P 29/00

(54) **PROTEIN WITH ACTIVITY OF INHIBITING NEOVASCULARIZATION GROWTH AND INHIBITING INFLAMMATORY RESPONSE, AND PREPARATION METHOD THEREFOR**

(30) Priority: 11.12.2020 CN 202011452867
(71) Applicant: Zhaoke (Guangzhou) Ophthalmology Pharmaceutical Ltd., Guangzhou, Guangdong 511466 (CN)
(72) Inventor: LI, Xiaoyi, Guangzhou, Guangdong 511466 (CN); DAI, Xiangrong, Guangzhou, Guangdong 511466 (CN); FANG, Li, Guangzhou, Guangdong 511466 (CN); QIAN, Fang, Guangzhou, Guangdong 511466 (CN); YAN, Yinping, Guangzhou, Guangdong 511466 (CN); DAI, Zicheng, Guangzhou, Guangdong 511466 (CN); LI, Luoyi, Guangzhou, Guangdong 511466 (CN)
(74) Representative: Isern Patentes y Marcas S.L.
(86) International application number: PCT/CN2021/071407
(87) International publication number: WO 2022/121084

(57) **Abstract**

The present disclosure discloses a protein with an activity of inhibiting angiogenesis and inflammation, and a specific structure, use, and preparation method thereof. The protein can inhibit both angiogenesis and inflammation, and can prevent and alleviate ophthalmic diseases such as age-related macular degeneration (AMD), diabetic retinopathy (DR), and pterygium to some extent based on a dual action mechanism.

## Description

### TECHNICAL FIELD

The present disclosure belongs to the field of biomedicine, and in particular relates to a protein with an activity of inhibiting angiogenesis and inflammation, and a preparation method thereof.

### BACKGROUND

Many ophthalmic diseases are clinically related to ocular tissue inflammation and pathological angiogenesis, including, but not limited to, wet age-related macular degeneration (AMD), diabetic retinopathy (DR), uveitis, and conjunctival connective tissue proliferation (pterygium). After an eye tissue is subjected to an external stimulus or damage, an inflammatory response occurs to increase the release of growth factors to repair a damaged tissue. Vascular endothelial growth factor (VEGF) is one of the important growth factors in the human body, and mainly plays a role of promoting the proliferation of vascular endothelial cells (VECs) and the angiogenesis. Angiogenesis is a primary mechanism of vascular growth during embryonic development and wound healing. A normal angiogenesis process includes not only endothelial cell (EC) migration and proliferation, but also vascular maturation, vascular remodeling, extracellular matrix (ECM) degradation, or the like. Under normal conditions, ECs are quiescent and do not proliferate due to the balance of expression levels and functions of angiogenesis factors such as VEGF and pigment epithelium-derived factor (PEDF). Pathological angiogenesis is significantly different from normal angiogenesis, and the excess secretion of VEGF can induce the overgrowth of ECs to rapidly produce uncontrolled pathological new blood vessels. These new blood vessels have fragile structures and are prone to bleeding and leakage. In addition, VEGF causes gaps among microvascular endothelial cells (MVECs), thereby increasing the permeability of blood vessels. If pathological angiogenesis occurs in a choroid, the pathological angiogenesis may invade a retina, exuded blood may lead to retinal macular degeneration, and patients with severe conditions may even lose vision. If angiogenesis occurs in a conjunctiva, pathological conjunctival tissue proliferation may be induced. An eye inflammation may be caused by external stimulation, infection, or accidental damage, and inflammations at different sites may cause different lesions, including keratitis and uveitis. Studies have shown that long-term inflammatory stimulation will increase the expression of VEGF and accelerate the pathological angiogenesis. Currently, the clinical treatment of angiogenesis-associated ophthalmic diseases is mainly conducted by monoclonal antibodies (mAbs) for VEGF clearance. Therefore, an anti-angiogenesis drug with anti-inflammatory bioactivity can provide excellent efficacy. In view of this, the present disclosure is specifically proposed.

### SUMMARY

The existing drug treatment for angiogenesis mainly focuses on the blockage of vascular leakage and the inhibition of angiogenesis, and clinical treatment drugs mainly include mAbs against VEGF. Although mAb drugs show specified efficacy, but have a risk of interfering with an immune system and inducing systemic inflammation. Moreover, mAb drugs have a large molecular weight and a complicated structure and are not easy to store, and more importantly, the production technique and environment of mAb drugs have high requirements, resulting in relatively-high prices of mAb drugs.

In view of this, the present disclosure provides a protein ZK002 with a bioactivity of inhibiting angiogenesis and inflammation, and a preparation method of the protein is simple, has a low cost, and is conducive to industrial production.

The present disclosure provides a protein ZK002 with an activity of inhibiting angiogenesis and inflammation, where a molecular weight of the protein is determined by non-reducing sodium dodecyl sulfate-polyacrylamide gel electrophoresis (SDS-PAGE) to be 25,000 Da to 35,000 Da; a molecule of the protein is composed of a chain α and a chain β, where the chain α has a protein sequence shown in SEQ ID No: 1 and the chain β has a protein sequence shown in SEQ ID No: 2; and the protein has an activity of inhibiting angiogenesis and inflammation.

Through reducing SDS-PAGE, a molecular weight of the chain α in the protein is determined to be 12,000 Da to 22,000 Da, and a molecular weight of the chain β in the protein is determined to be 9,000 Da to 19,000 Da. By observing a crystal structure of the protein molecule, it can be known that there are 6 pairs of intrachain disulfide bonds (with 3 pairs in each of the chains α and β) and 1 pair of interchain disulfide bonds in the protein molecule.

Further, the present disclosure also discloses a use of the protein in inhibition of angiogenesis and inflammation and a use of the protein in a pharmaceutical composition. Further, the pharmaceutical composition further includes a pharmaceutically acceptable carrier or a pharmaceutically acceptable excipient and/or a stabilizer.

Further, the present disclosure also discloses a preparation method of the protein ZK002, including the following steps sequentially:
(1) dissolving venom of *Deinagkistrodon acutus* (*D. acutus*) with a Tris-HCl buffer, centrifuging, and collecting a resulting supernatant;
(2) subjecting the supernatant obtained in step (1) to adsorption with a fully-equilibrated anion exchange chromatography column, and after the adsorption is completed, eluting unabsorbed impurity protein with a solution A; and after the eluting with the solution A is completed, conducting gradient elution with a solution B, collecting an elution product by a fraction collector, and detecting and tracking a peak of each component at 280 nm by an ultraviolet (UV) detector, where the solution A is a 0.01 mol/L to 0.1 mol/L Tris-HCl solution with a pH of 7.0 to 9.0, and the solution B is a mixed solution of 0.01 mol/L to 0.05 mol/L Tris-HCl and 0.05 mol/L to 0.6 mol/L NaCl that has a pH of 6.0 to 9.0;
(3) subjecting an eluted target protein product collected in step (2) to ultrafiltration concentration by a membrane system with a molecular weight cut-off (MWCO) of 3,000 Da to 10,000 Da, and subjecting a resulting concentrate to adsorption with a fully-equilibrated cation exchange chromatography column; after the adsorption is completed, eluting unabsorbed impurity protein with a solution C; and after the eluting with the solution C is completed, conducting gradient elution with a solution D, collecting an elution product by a fraction collector, and detecting and tracking a peak of each component at 280 nm by a UV detector, where the solution C is a 0.01 mol/L to 0.1 mol/L Tris-HCl solution with a pH of 6.0 to 9.0, and the solution D is a mixed solution of 0.01 mol/L to 0.06 mol/L Tris-HCl and 0.05 mol/L to 0.6 mol/L NaCl that has a pH of 7.0 to 9.0.
(4) subjecting an eluted target protein product collected in step (3) to ultrafiltration concentration by a membrane system with an MWCO of 3,000 Da to 10,000 Da, and subjecting a resulting concentrate to adsorption with an affinity chromatography column; after the adsorption is completed, equilibrating the affinity chromatography column with an equilibration buffer; and eluting with a Gly buffer, collecting an elution product corresponding to a Gly elution peak, and adjusting a pH of the elution product with NaOH to 6.0 to 9.0, where the equilibration buffer is a mixed solution of 0.01 mol/L to 0.1 mol/L Tris-HCl and 0.05 mol/L to 0.5 mol/L NaCl that has a pH of 6.0 to 9.0, and the Gly buffer is 0.1 mol/L to 0.5 mol/L Gly with a PH of 2.0 to 4.0; and
(5) subjecting an elution product obtained after the pH adjustment in step (4) to ultrafiltration concentration by a filter membrane with an MWCO of 3,000 Da to 10,000 Da, and subjecting a resulting concentrate to adsorption with a gel chromatography column; and after the adsorption is completed, eluting with a solution E, and collecting an elution product corresponding to an elution peak, which is a purified protein product, where the solution E is a mixed solution of 0.01 mol/LM to 0.1 mol/LM Tris-HCl and 0.05 mol/L to 0.5 mol/L NaCl that has a pH of 6.0 to 9.0.

Preferably, the Tris-HCl buffer used in step (1) has a pH of 7.0 to 9.0 and a concentration of 0.01 mol/L to 0.1 mol/L.

Preferably, the dissolving in step (1) is conducted at 4°C for 6 h to 12 h.

Preferably, the centrifuging in step (1) is conducted at 4,000 r/min twice for 10 min each time.

Preferably, the adsorption of the supernatant in step (2) is conducted at a flow rate of 50 mL/min.

Preferably, in step (2), the eluting with the solution A is conducted at a flow rate of 48 mL/min for 600 min; and the gradient elution with the solution B is conducted at a flow rate of 43 mL/min.

Preferably, the adsorption in step (3) is conducted at a flow rate of 5 mL/min.

Preferably, in step (3), the eluting with the solution C is conducted at a flow rate of 5.5 mL/min for 300 min; and the gradient elution with the solution D is conducted at a flow rate of 5.5 mL/min.

Preferably, the equilibrating with the equilibration buffer in step (4) is conducted at a flow rate of 0.5 mL/min.

Preferably, the adsorption in step (5) is conducted at a flow rate of 2.0 mL/min.

Preferably, the eluting with the solution E in step (5) is conducted at a flow rate of 0.3 mL/min.

### Compared with the prior art, the present disclosure has the following advantages:

The natural protein ZK002 disclosed in the present disclosure can inhibit both angiogenesis and inflammation, and can prevent and alleviate ophthalmic diseases such as AMD, DR, and pterygium to some extent based on a dual action mechanism. In addition, an extraction and purification method of ZK002 is relatively simple, and has strong repeatability and low cost, which is conducive to industrial production.

Animal experimental results show that, ZK002 exhibits a stronger inhibitory effect for corneal angiogenesis than the existing anti-angiogenesis drug conbercept.

### BRIEF DESCRIPTION OF THE DRAWINGS

FIG. 1 is an absorption spectrum for elution with a solution B in step (2) in Example 1 of the present disclosure;
FIG. 2 is an absorption spectrum for elution with a solution D in step (3) in Example 1 of the present disclosure;
FIG. 3 is an absorption spectrum for elution with a Gly buffer in step (4) in Example 1 of the present disclosure;
FIG. 4 is an absorption spectrum for elution with a solution E in step (5) in Example 1 of the present disclosure;
FIG. 5 shows an impact of ZK002 on the production of NO in Example 2 of the present disclosure;
FIG. 6 shows an impact of ZK002 on the expression of iNOS in Example 2 of the present disclosure;
FIG. 7 shows an impact of ZK002 on the mRNA expression of pro-inflammatory factors and a tumor necrosis factor (TNF)-a in Example 2 of the present disclosure;
FIG. 8 shows an impact of ZK002 on the mRNA expression of lipopolysaccharide (LPS)-induced pro-inflammatory factors IL-6 and IL-8 in Example 3 of the present disclosure;
FIG. 9 shows an impact of ZK002 on the mRNA expression of LPS-induced pro-inflammatory factors IL-1β and IL-6 and TNF-a in Example 4 of the present disclosure;
FIG. 10 shows an impact of ZK002 on the transfer of LPS-induced NF-κB from cytoplasm to nucleus in Example 4 of the present disclosure;
FIG. 11 shows an alleviation effect of ZK002 for carrageenan-induced swelling of a rat paw in Example 5 of the present disclosure;
FIG. 12 shows an alleviation effect of ZK002 for collagen-induced arthritis of a mouse in Example 5 of the present disclosure;
FIG. 13 shows an inhibitory effect of ZK002 for fetal bovine serum (FBS)-induced human umbilical vein endothelial cell (HUVEC) tube formation in Example 6 of the present disclosure;
FIG. 14 shows angiogenesis results of stained retinas in Example 7 of the present disclosure;
FIG. 15 shows retinal thickness results in Example 7 of the present disclosure;
FIG. 16 shows an inhibitory effect of ZK002 on inflammation-associated cytokines and chemokines in Example 7 of the present disclosure;
FIG. 17 shows angiogenesis results of stained retinas in Example 8 of the present disclosure;
FIG. 18 shows retinal thickness results in Example 8 of the present disclosure;
FIG. 19 shows an inhibitory effect of ZK002 on inflammation-associated cytokines and chemokines in Example 8 of the present disclosure;
FIG. 20 shows angiogenesis results of stained retinas in Example 9 of the present disclosure;
FIG. 21 shows retinal thickness results in Example 9 of the present disclosure;
FIG. 22 shows an inhibitory effect of ZK002 on inflammation-associated cytokines and chemokines in Example 9 of the present disclosure;
FIG. 23 shows contrast images illustrating angiogenesis conditions of cauterized rat corneas in Example 10 of the present disclosure; and
FIG. 24 shows angiogenesis conditions of cauterized rat corneas in Example 10 of the present disclosure.

### DETAILED DESCRIPTION OF THE EMBODIMENTS

In order to clearly explain the technical solutions of the present disclosure, the technical solutions of the present disclosure will be further described below with reference to specific embodiments.

### Example 1 Preparation of a protein ZK002

In this example, a specific preparation method of the protein ZK002 was provided.

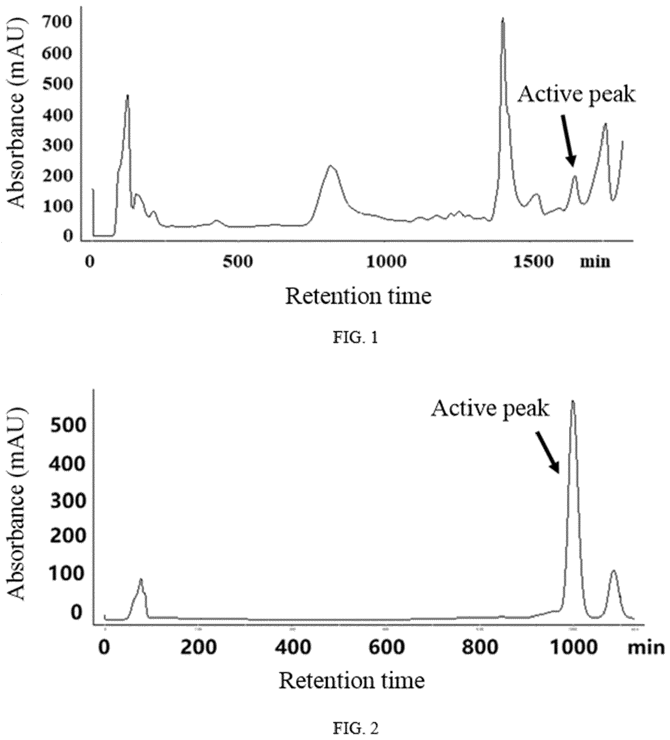

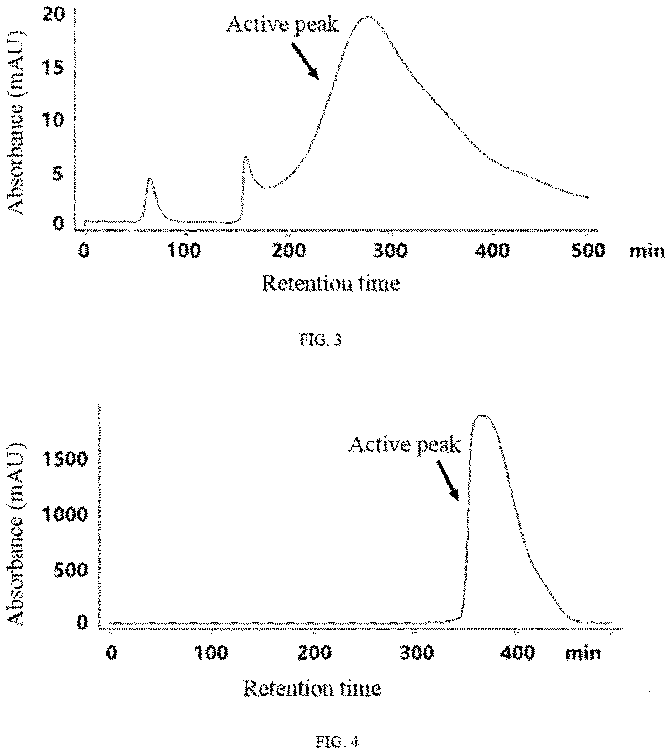

The method included the following steps sequentially:
(1) 40 g of venom of *D. acutus* was weighed and dissolved with 320 mL of a 0.02 M pH 8.0 Tris-HCl buffer in a 4°C refrigerated environment for 6 h to 12 h, a resulting solution was continuously centrifuged at 4,000 r/min twice for 10 min each time, and a resulting supernatant was collected.
(2) The supernatant obtained in step (1) was loaded at a flow rate of 50 mL/min into a fully-equilibrated anion exchange chromatography column DEAE for adsorption, where the column DEAE had a model of BXK200×500 and a volume of 8 L, and the supernatant was loaded in a volume of 200 mL or less during the adsorption. After the adsorption was completed, impurity protein that were not adsorbed by a chromatography medium were eluted with a solution A (0.02 M Tris-HCl, pH 8.0) at a flow rate of 48 mL/min for 600 min, and then gradient elution was conducted with a solution B (0.02 M Tris-HCl + 0.3 M NaCl, pH 6.0) at a flow rate of 43 mL/min. During the gradient elution with the solution B, a component was detected at 280 nm by a UV detector of a purification system AKTA Pilot and collected by a fraction collector of the system. An absorption spectrum for the elution with the solution B was shown in FIG. 1. An elution product corresponding to a target peak was collected for a subsequent step.
(3) The elution product collected in step (2) was subjected to ultrafiltration concentration by a filter membrane with an MWCO of 5,000 Da until a volume of a resulting concentrate was 100 mL to 200 mL, and the concentrate was loaded at a flow rate of 5 mL/min into a fully-equilibrated cation exchange chromatography column for adsorption, where the chromatography column had a model of XK50×60 and a volume of 1 L. After the adsorption was completed, impurity protein that were not adsorbed by a chromatography medium were eluted with a solution C (0.02 M Tris-HCl, pH 6.0) at a flow rate of 5.5 mL/min for 300 min, and then gradient elution was conducted with a solution D (0.02 M Tris-HCl + 0.5 M NaCl, pH 8.0) at a flow rate of 5.5 mL/min. During the gradient elution with the solution D, a component was detected at 280 nm by a UV detector of a purification system AKTA prime plus and collected by a fraction collector of the system. An absorption spectrum for the elution with the solution D was shown in FIG. 2. An elution product corresponding to a target peak was collected for a subsequent step.
(4) The elution product of peak 2 collected in step (2) was subjected to ultrafiltration concentration by a filter membrane with an MWCO of 5,000 Da until a volume of a resulting concentrate was 30 mL or less, and the concentrate was loaded into a negative affinity chromatography column for adsorption, where the negative affinity chromatography column had a model of XK16×40 and a volume of 60 mL. After the adsorption was completed, impurity protein that were not adsorbed by a chromatography medium was eluted with an equilibration buffer (0.02 M Tris-HCl + 0.15 M NaCl, pH 7.6) at a flow rate of 0.5 mL/min, and then elution was conducted with a Gly buffer. An absorption spectrum for the elution with the Gly buffer was shown in FIG. 3. An elution product corresponding to a target peak was collected, and a pH of the collected elution product was immediately adjusted with 1 M NaOH to 7.0.
(5) The elution product of an active peak collected in step (4) was subjected to ultrafiltration concentration by a filter membrane with an MWCO of 5,000 Da until a volume of a resulting concentrate was 5% or less of a volume of the chromatography column, and the concentrate was loaded at a flow rate of 2.0 mL/min into a gel chromatography column for adsorption, where the gel chromatography column had a model of XK26×100cm and a volume of 0.5 L. After the adsorption was completed, elution was conducted with a solution E (0.02 M Tris-HCl + 0.15 M NaCl, pH 7.6) at a flow rate of 0.3 mL/min. An absorption spectrum for the elution was shown in FIG. 4. An elution product corresponding to a target peak was collected, which was a purified target protein.

### Example 2 Evaluation of an inhibitory effect of ZK002 for inflammation by a human retinal pigment epithelium (RPE) cell model

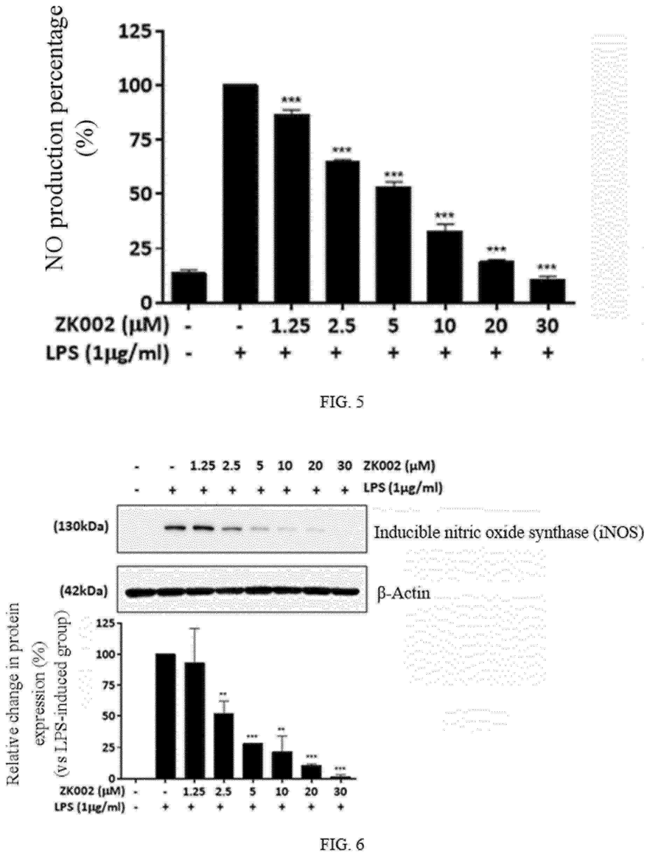

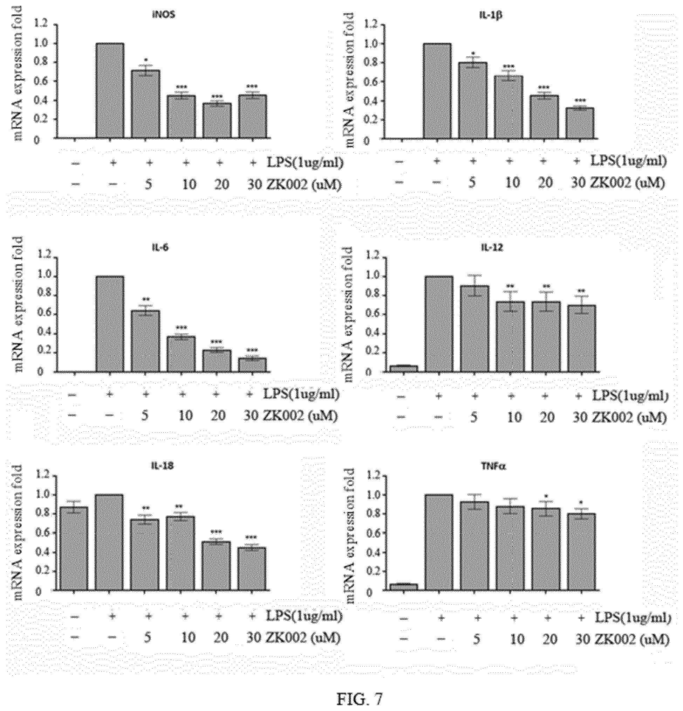

A human RPE cell (APRE19) was adopted as an *in vitro* model, and an LPS was added at a concentration of 1 µg/mL to a cultivation environment of the APRE19 cell to induce inflammation of the APRE19 cell, which was specifically manifested as follows: the expression of inducible nitric oxide synthase (iNOS) and the production of nitric oxide (NO) were increased, and the mRNA expression of some representative pro-inflammatory factors including iNOS, interleukin (IL)-1β, IL-6, IL-12, and IL-18 and TNF-α was increased significantly. The protein ZK002 was added at different concentrations (1.25 µM to 30 µM) together with LPS to the cultivation environment. Experimental results were shown in FIG. 5 to FIG. 7 and Tables 1 and 2.

**Table 1 Inhibitory effect of the protein ZK002 for the iNOS expression and NO production in the LPS-induced APRE19 cell**

| ZK002 (µM) | NO production (%) | iNOS expression (%) |
|---|---|---|
| 1.25 | 86.80 | 93.10 |
| 2.5 | 65.60 | 51.80 |
| 5 | 53.40 | 28.20 |
| 10 | 33.10 | 21.50 |
| 20 | 18.90 | 10.60 |
| 30 | 10.88 | 1.70 |
| LPS (1 µg/mL) | 100 | 100 |

**Table 2 Inhibitory effect of the protein ZK002 for the mRNA expression of pro-inflammatory factors in the LPS-induced APRE19 cell**

| ZKOO2 (µM) | mRNA expression of iNOS (%) | mRNA expression of IL-1β (%) | mRNA expression of IL-6 (%) | mRNA expression of IL-12 (%) | mRNA expression of IL-18 (%) | mRNA expression of TNF-α (%) |
|---|---|---|---|---|---|---|
| 5 | 71.4 | 80.2 | 64.3 | 89.9 | 74.0 | 92.4 |
| 10 | 45.0 | 66.2 | 36.6 | 73.3 | 77.2 | 87.8 |
| 20 | 36.6 | 45.3 | 22.7 | 73.1 | 51.2 | 86.0 |
| 30 | 45.2 | 32.3 | 14.2 | 69.8 | 45.0 | 80.0 |
| LPS (1 µg/mL) | 100 | 100 | 100 | 100 | 100 | 100 |

It can be seen from the results shown in FIG. 5 to FIG. 7 and Tables 1 and 2 that the protein ZK002 can inhibit the iNOS expression and NO production and the mRNA expression of various pro-inflammatory factors in the LPS-induced APRE19 cell in a concentration-dependent manner, that is, the protein ZK002 has a specified inhibitory effect for inflammation.

### Example 3 Evaluation of an inhibitory effect of ZK002 for inflammation by an HUVEC model

An HUVEC was adopted as an *in vitro* model, and LPS was added at a concentration of 1 µg/mL to a cultivation environment of the HUVEC cell to induce inflammation of the HUVEC cell, which was specifically manifested as follows: LPC could induce a significant increase in the mRNA expression of pro-inflammatory factors IL-6 and IL-8.

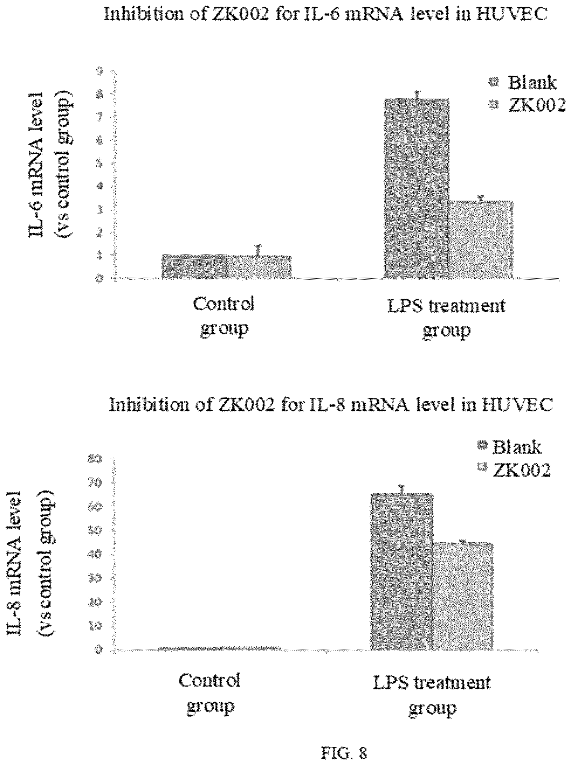

The protein ZK002 (5 µM) was added to the HUVEC cell, then the HUVEC cell was pre-cultivated for 24 h, and then LPS (1 µg/mL) was added. Experimental results were shown in FIG. 8.

It can be seen from the results in FIG. 8 that the protein ZK002 has a specified inhibitory effect for the LPS-induced mRNA expression of pro-inflammatory factors IL-6 and IL-8, that is, the protein ZK002 has a specified inhibitory effect for inflammation.

### Example 4 Evaluation of an inhibitory effect of ZK002 for inflammation by a mouse macrophage model

A mouse macrophage (RAW264.7) was adopted as an *in vitro* model, and LPS was added at a concentration of 1 µg/mL to a cultivation environment of the RAW264.7 cell to induce inflammation of the RAW264.7 cell, which was specifically manifested as follows: LPC could induce a significant increase in the mRNA expression of pro-inflammatory factors IL-1β and IL-6 and TNF-a.

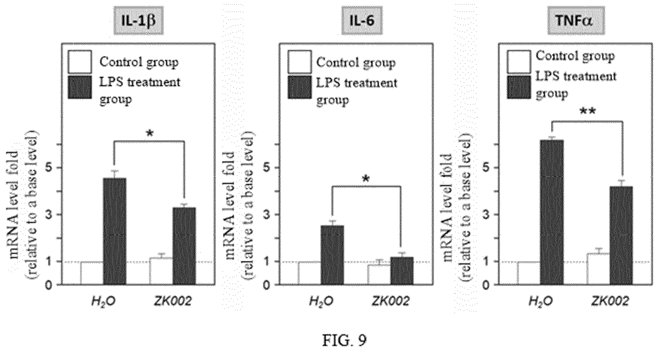

The protein ZK002 (5 µM) was added to the RAW264.7 cell, then the RAW264.7 cell was pre-cultivated for 24 h, and then LPS (1 µg/mL) was added. Experimental results were shown in FIG. 9.

It can be seen from the results in FIG. 9 that the protein ZK002 has a specified inhibitory effect for the LPS-induced mRNA expression of pro-inflammatory factors IL-1β and IL-6 and TNF-a.

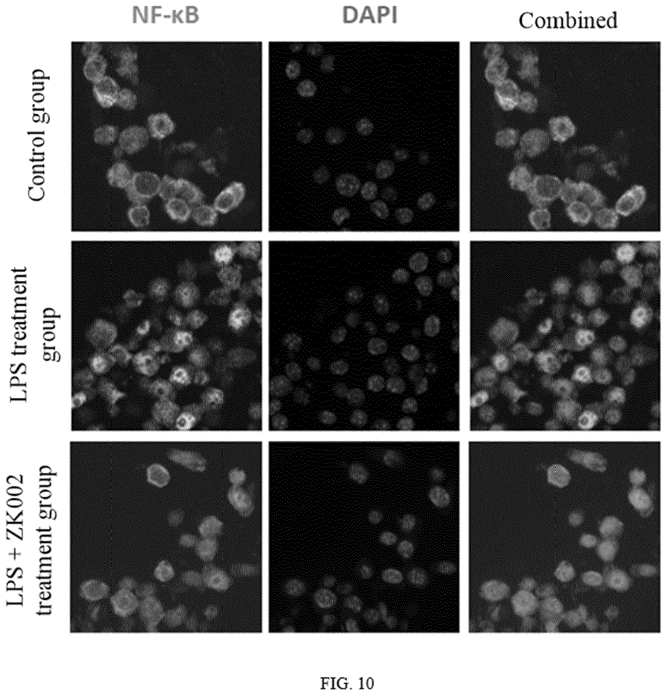

In addition, LPS could induce an increase in the transfer of NF-κB from cytoplasm to nucleus, and after the treatment with the protein ZK002, the increase in the transfer of NF-κB from cytoplasm to nucleus induced by LPS was inhibited. Experimental results were shown in FIG. 10.

In summary, the protein ZK002 can inhibit the inflammation of cells and has a specified anti-inflammatory effect.

### Example 5 Evaluation of an inhibitory effect of ZK002 for inflammation by intact animal models

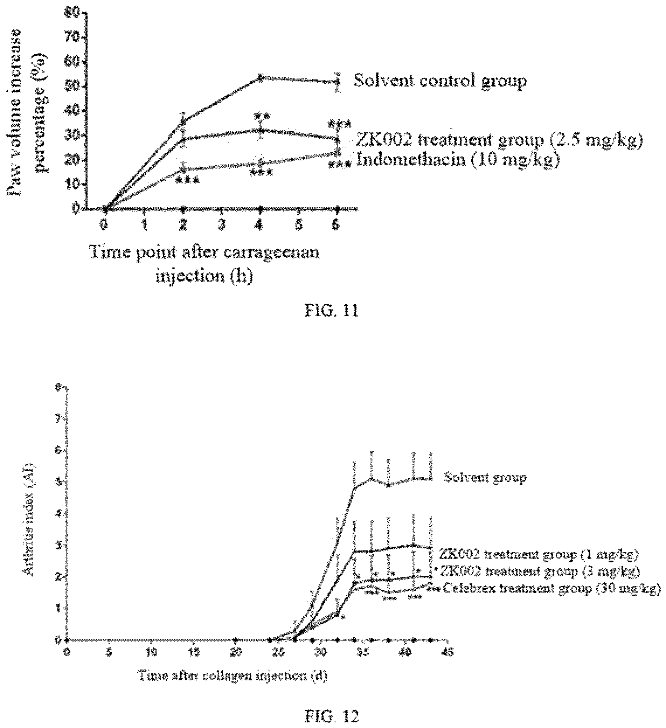

An SPF rat and an SPF mouse were adopted as intact animal models. A hind paw of the rat was subcutaneously injected with 100 µL of 1% carrageenan to simulate acute inflammation, and 4 h after the injection, a size of the paw of the rat was increased by 50%. The rat was intraperitoneally injected with the protein ZK002 (2.5 mg/kg) 1 h before the inflammation simulation. A volume of the hind foot of the rat was measured at different time points before and after the inflammation simulation, and results were shown in FIG. 11.

It can be seen from the results of FIG. 11 that ZK002 (2.5 mg/kg) can alleviate the carrageenan-induced swelling of the paw with a similar effect to indomethacin (10 mg/kg).

The mouse was injected with collagen to induce arthritis, thereby simulating long-term inflammation. The mouse was intraperitoneally injected with the protein ZK002 (1 mg/kg and 3 mg/kg), and experimental results were shown in FIG. 12.

It can be seen from the results of FIG. 12 that ZK002 can significantly alleviate the collagen-induced arthritis, and the efficacy of ZK002 (3 mg/kg) is similar to the efficacy of Celebrex (30 mg/kg).

The above results show that the protein ZK002 has a specified alleviation effect for both acute and long-term inflammation.

### Example 6 Evaluation of an inhibitory effect of ZK002 for angiogenesis by an HUVEC model

An HUVEC was adopted as an *in vitro* model. The HUVEC could be inoculated on a Matrigel matrix, 2% FBS was added, and the HUVEC was cultivated for 8 h to 12 h to produce a tubular structure.

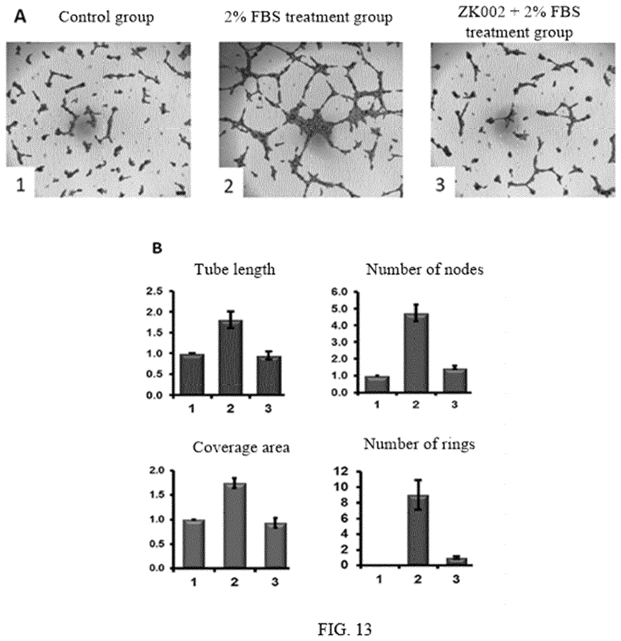

The protein ZK002 (3 µM) and 2% FBS were added to the HUVEC, and the HUVEC was further cultivated for 8 h. Experimental results were shown in FIG. 13.

It can be seen from FIG. 13 that the addition of ZK002 can significantly inhibit a total length, a number of nodes, a coverage area, and a number of rings for FBS-induced production of tubular structures from the HUVEC, that is, the protein ZK002 has a specified inhibitory effect for angiogenesis.

### Example 7 Evaluation of an inhibitory effect of ZK002 for angiogenesis, inflammation, and DR by an intact animal model

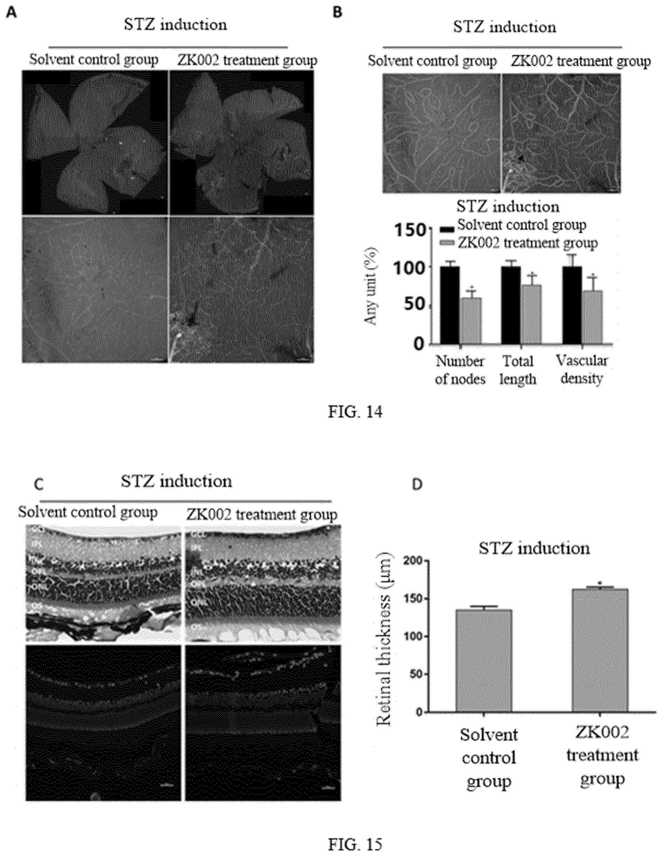

8-week-old male C57BL/6 mice were taken as an intact animal model, and the mice were intraperitoneally injected with streptozotocin (STZ) (150 mg/kg) once to cause a functional impairment of pancreatic islet β cells, thereby increasing a blood glucose level in the mice. When a blood glucose level in a mouse was higher than 18 mmol/L, the mouse developed type I diabetes. The mice were randomly divided into two groups (with 10 in each group), and vitreous bodies of the mice in the two groups were injected with normal saline (NS) and ZK002 (5 µg), respectively, where the administration was conducted twice a week for two weeks. 14 d later, an eyeball was removed, a retina was fixed and subjected to immunofluorescence (IF) staining with a fluorescein isothiocyanate (FITC)-conjugated antibody or hematoxysin-eosin (H&E) staining, and the angiogenesis and retinal thickness change were observed under a fluorescence microscope. Experimental results were shown in FIG. 14 and FIG. 15.

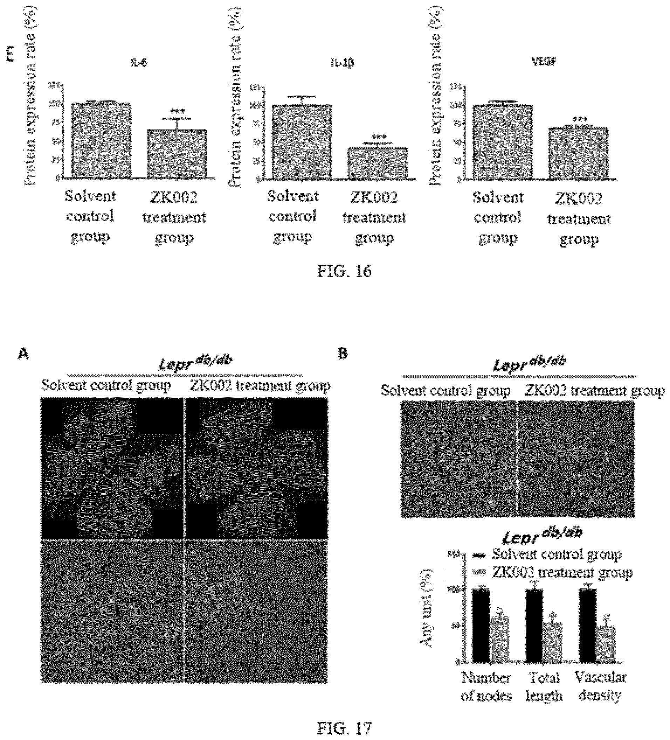

Further, a retinal sample was collected and homogenized, and then the changes in inflammation-associated cytokines and chemokines were determined by enzyme-linked immunosorbent assay (ELISA). Experimental results were shown in FIG. 16.

It can be seen from FIG. 14 and FIG. 15 that the protein ZK002 can significantly inhibit the retinal angiogenesis while reducing a degree of retinal thinning.

It can be seen from FIG. 16 that the protein ZK002 can inhibit the expression of retinal pro-inflammatory factors in diabetic mice.

It can be seen that the protein ZK002 has a specified inhibitory effect for angiogenesis and inflammation, and also has a specified potential for preventing and alleviating retinopathy caused by diabetes.

### Example 8 Evaluation of an inhibitory effect of ZK002 for angiogenesis, inflammation, and DR by an intact animal model

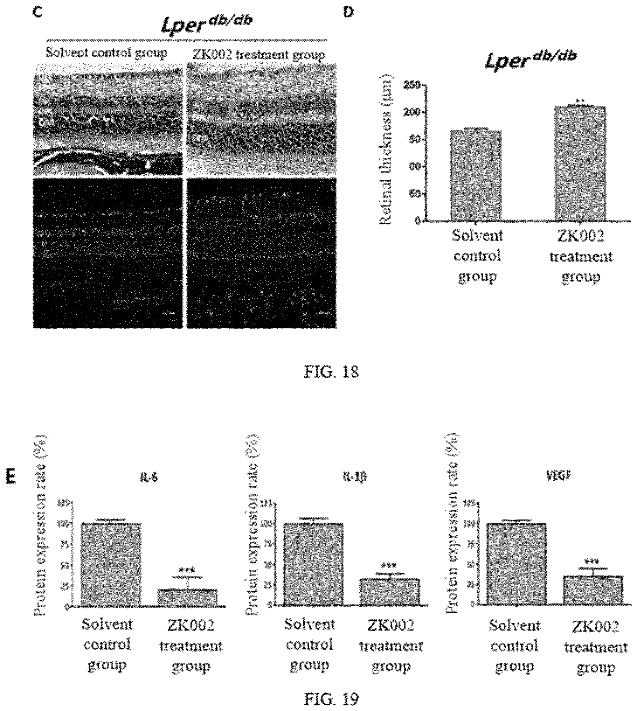

16-week-old Leprdb/db transgenic adult mice with type II diabetes were adopted as an intact animal model. The mice were randomly divided into two groups (with 10 in each group), and vitreous bodies of the mice in the two groups were injected with NS and ZK002 (5 µg), respectively, where the administration was conducted twice a week for two weeks. 14 d later, an eyeball was removed, a retina was fixed and subjected to IF staining with an FITC-conjugated antibody or H&E staining, and the angiogenesis and retinal thickness change were observed under a fluorescence microscope. Experimental results were shown in FIG. 17 and FIG. 18.

Further, a retinal sample was collected and homogenized, and then the changes in inflammation-associated cytokines and chemokines were determined by ELISA. Experimental results were shown in FIG. 19.

It can be seen from FIG. 17 and FIG. 18 that the protein ZK002 can significantly inhibit the retinal angiogenesis while reducing a degree of retinal thinning.

It can be seen from FIG. 19 that the protein ZK002 can inhibit the expression of retinal pro-inflammatory factors in diabetic mice.

It can be seen that the protein ZK002 has a specified inhibitory effect for angiogenesis and inflammation, and also has a potential for preventing and alleviating retinopathy caused by diabetes.

### Example 9 Evaluation of an inhibitory effect of ZK002 for angiogenesis, inflammation, and DR by an intact animal model

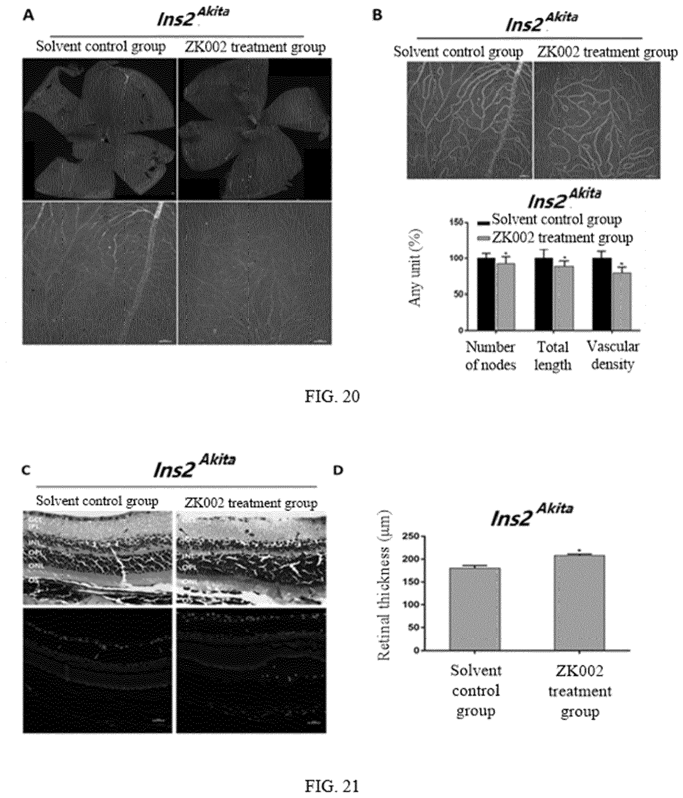

16-week-old Ins2Akita transgenic adult mice with type I diabetes were adopted as an intact animal model. The mice were randomly divided into two groups (with 10 in each group), and vitreous bodies of the mice in the two groups were injected with NS and ZK002 (5 µg), respectively, where the administration was conducted twice a week for two weeks. 14 d later, an eyeball was removed, a retina was fixed and subjected to IF staining with an FITC-conjugated antibody or H&E staining, and the angiogenesis and retinal thickness change were observed under a fluorescence microscope. Experimental results were shown in FIG. 20 and FIG. 21.

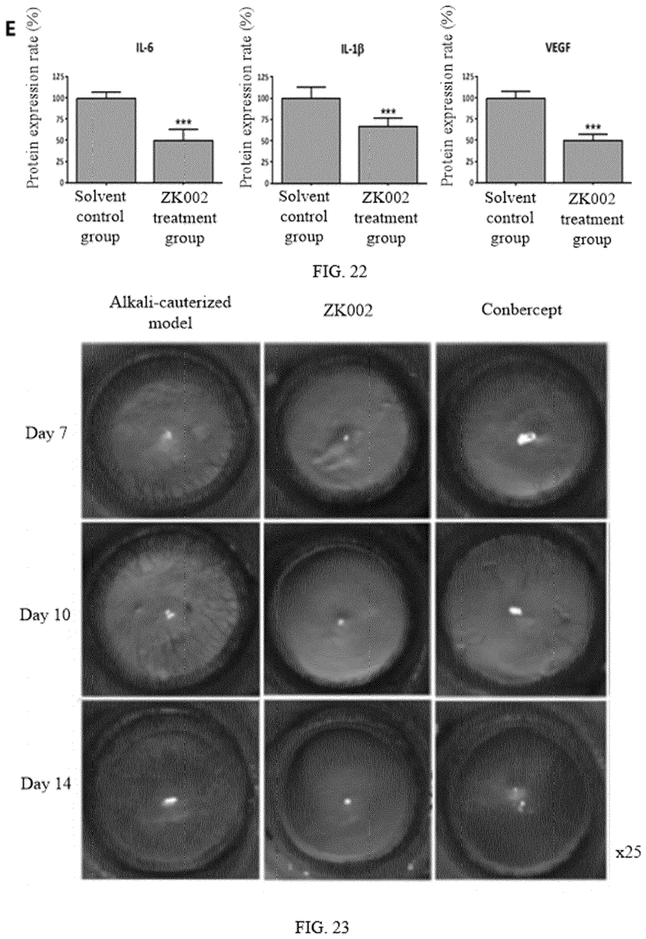

Further, a retinal sample was collected and homogenized, and then the changes in inflammation-associated cytokines and chemokines were determined by ELISA. Experimental results were shown in FIG. 22.

It can be seen from FIG. 20 and FIG. 21 that the protein ZK002 can significantly inhibit the retinal angiogenesis while reducing a degree of retinal thinning.

It can be seen from FIG. 22 that the protein ZK002 can inhibit the expression of retinal pro-inflammatory factors in diabetic mice.

It can be seen that the protein ZK002 has a specified inhibitory effect for angiogenesis and inflammation, and also has a potential for protecting and treating retinopathy caused by diabetes.

### Example 10 Evaluation of an inhibitory effect of ZK002 for corneal angiogenesis by an intact animal model

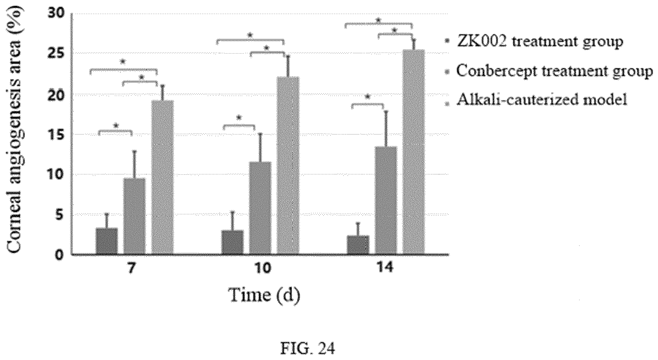

8-12-week-old SD rats were adopted as an intact animal model. The rats were randomly divided into three groups (with 10 rats in each group), and the right corneas of the rats were cauterized by a filter paper infiltrated with 1 mol/L sodium hydroxide. A contralateral eye was taken as a blank control. The administration was started on the day of modeling. Rats in each group received a conbercept (1.5 mg/mL) or ZK002 (1.5 mg/mL) eye drop three times a day for two weeks. The angiogenesis was observed on day 7, day 10, and day 14, and results were shown in FIG. 23 and FIG. 24.

It can be seen from FIG. 23 and FIG. 24 that ZK002 can significantly inhibit the corneal angiogenesis, with a more significant inhibitory effect than the conbercept as a positive control group.

The present disclosure provides a protein ZK002 and a specific feasible preparation method thereof. The protein ZK002 has a specified inhibitory effect for angiogenesis and inflammation, has a potential for preventing and alleviating retinopathy caused by diabetes, and can improve the efficacy for ophthalmic diseases such as AMD, DR, and pterygium based on a dual action mechanism. The protein ZK002 has a better effect than the existing similar drugs (such as mAb against VEGF); and an extraction and purification method of the protein is relatively simple, and has strong repeatability and low cost, which is conducive to industrial production.

The above are merely preferred specific embodiments of the present disclosure, but the protection scope of the present disclosure is not limited thereto. Any equivalent replacement or modification made by a person skilled in the art within the technical scope of the present disclosure according to the technical solutions of the present disclosure and inventive concepts thereof shall fall within the protection scope of the present disclosure.

## Claims

1. A protein with an activity of inhibiting angiogenesis and inflammation, wherein a molecular weight of the protein is determined by non-reducing sodium dodecyl sulfate-polyacrylamide gel electrophoresis (SDS-PAGE) to be 25,000 Da to 35,000 Da; a molecule of the protein is composed of a chain α and a chain β, wherein the chain α has a protein sequence shown in SEQ ID No: 1 and the chain β has a protein sequence shown in SEQ ID No: 2; and the protein has an activity of inhibiting angiogenesis and inflammation.

2. The protein with the activity of inhibiting the angiogenesis and the inflammation according to claim 1, wherein through reducing SDS-PAGE, a molecular weight of the chain α is determined to be 12,000 Da to 22,000 Da, and a molecular weight of the chain β is determined to be 9,000 Da to 19,000 Da.

3. A use of the protein according to any one of claims 1 and 2 in inhibition of the angiogenesis and the inflammation.

4. A use of the protein with the activity of inhibiting the angiogenesis and the inflammation according to any one of claims 1 and 2 in a pharmaceutical composition.

5. The use of the protein with the activity of inhibiting the angiogenesis and the inflammation in a pharmaceutical composition according to claim 4, wherein the pharmaceutical composition further comprises a pharmaceutically acceptable carrier or a pharmaceutically acceptable excipient and/or a stabilizer.

6. A preparation method of the protein with the activity of inhibiting the angiogenesis and the inflammation according to any one of claims 1 and 2, comprising the following steps sequentially:
(1) dissolving venom of *Deinagkistrodon acutus* (*D. acutus*) with a Tris-HCl buffer, centrifuging, and collecting a resulting supernatant;
(2) subjecting the supernatant obtained in step (1) to adsorption with a fully-equilibrated anion exchange chromatography column, and after the adsorption is completed, eluting unabsorbed impurity protein with a solution A; and after the eluting with the solution A is completed, conducting gradient elution with a solution B, collecting an elution product by a fraction collector, and detecting and tracking a peak of each component at 280 nm by an ultraviolet (UV) detector, wherein the solution A is a 0.01 mol/L to 0.1 mol/L Tris-HCl solution with a pH of 7.0 to 9.0, and the solution B is a mixed solution of 0.01 mol/L to 0.05 mol/L Tris-HCl and 0.05 mol/L to 0.6 mol/L NaCl that has a pH of 6.0 to 9.0;
(3) subjecting an eluted target protein product collected in step (2) to ultrafiltration concentration by a membrane system with a molecular weight cut-off (MWCO) of 3,000 Da to 10,000 Da, and subjecting a resulting concentrate to adsorption with a fully-equilibrated cation exchange chromatography column; after the adsorption is completed, eluting unabsorbed impurity protein with a solution C; and after the eluting with the solution C is completed, conducting gradient elution with a solution D, collecting an elution product by a fraction collector, and detecting and tracking a peak of each component at 280 nm by a UV detector, wherein the solution C is a 0.01 mol/L to 0.1 mol/L Tris-HCl solution with a pH of 6.0 to 9.0, and the solution D is a mixed solution of 0.01 mol/L to 0.06 mol/L Tris-HCl and 0.05 mol/L to 0.6 mol/L NaCl that has a pH of 7.0 to 9.0.
(4) subjecting an eluted target protein product collected in step (3) to ultrafiltration concentration by a membrane system with an MWCO of 3,000 Da to 10,000 Da, and subjecting a resulting concentrate to adsorption with an affinity chromatography column; after the adsorption is completed, equilibrating the affinity chromatography column with an equilibration buffer; and eluting with a Gly buffer, collecting an elution product corresponding to a Gly elution peak, and adjusting a pH of the elution product with NaOH to 6.0 to 9.0, wherein the equilibration buffer is a mixed solution of 0.01 mol/L to 0.1 mol/L Tris-HCl and 0.05 mol/L to 0.5 mol/L NaCl that has a pH of 6.0 to 9.0, and the Gly buffer is 0.1 mol/L to 0.5 mol/L Gly with a PH of 2.0 to 4.0; and
(5) subjecting an elution product obtained after the pH adjustment in step (4) to ultrafiltration concentration by a filter membrane with an MWCO of 3,000 Da to 10,000 Da, and subjecting a resulting concentrate to adsorption with a gel chromatography column; and after the adsorption is completed, eluting with a solution E, and collecting an elution product corresponding to an elution peak, which is a purified protein product, wherein the solution E is a mixed solution of 0.01 mol/L to 0.1 mol/L Tris-HCl and 0.05 mol/L to 0.5 mol/L NaCl that has a pH of 6.0 to 9.0.

7. The preparation method of the protein with the activity of inhibiting the angiogenesis and the inflammation according to claim 6, wherein the Tris-HCl buffer used in step (1) has a pH of 7.0 to 9.0 and a concentration of 0.01 mol/L to 0.1 mol/L.

8. The preparation method of the protein with the activity of inhibiting the angiogenesis and the inflammation according to claim 6, wherein the dissolving in step (1) is conducted at 4°C for 6 h to 12 h.

9. The preparation method of the protein with the activity of inhibiting the angiogenesis and the inflammation according to claim 6, wherein the centrifuging in step (1) is conducted at 4,000 r/min twice for 10 min each time.

10. The preparation method of the protein with the activity of inhibiting the angiogenesis and the inflammation according to claim 6, wherein the adsorption of the supernatant in step (2) is conducted at a flow rate of 50 mL/min.

11. The preparation method of the protein with the activity of inhibiting the angiogenesis and the inflammation according to claim 6, wherein in step (2), the eluting with the solution A is conducted at a flow rate of 48 mL/min for 600 min; and the gradient elution with the solution B is conducted at a flow rate of 43 mL/min.

12. The preparation method of the protein with the activity of inhibiting the angiogenesis and the inflammation according to claim 6, wherein the adsorption in step (3) is conducted at a flow rate of 5 mL/min.

13. The preparation method of the protein with the activity of inhibiting the angiogenesis and the inflammation according to claim 6, wherein in step (3), the eluting with the solution C is conducted at a flow rate of 5.5 mL/min for 300 min; and the gradient elution with the solution D is conducted at a flow rate of 5.5 mL/min.

14. The preparation method of the protein with the activity of inhibiting the angiogenesis and the inflammation according to claim 6, wherein the equilibrating with the equilibration buffer in step (4) is conducted at a flow rate of 0.5 mL/min.

15. The preparation method of the protein with the activity of inhibiting the angiogenesis and the inflammation according to claim 6, wherein the adsorption in step (5) is conducted at a flow rate of 2.0 mL/min.

16. The preparation method of the protein with the activity of inhibiting the angiogenesis and the inflammation according to claim 6, wherein the eluting with the solution E in step (5) is conducted at a flow rate of 0.3 mL/min.
